(19) Europäisches Patentamt
European Patent Office
Office européen des brevets

(11) **EP 4 425 163 A1**

(12) **EUROPEAN PATENT APPLICATION**
published in accordance with Art. 153(4) EPC

(43) Date of publication:
**04.09.2024 Bulletin 2024/36**

(21) Application number: **22886783.4**

(22) Date of filing: **18.10.2022**

(51) International Patent Classification (IPC):
**G01N 27/327** (2006.01)   **G01N 27/416** (2006.01)

(52) Cooperative Patent Classification (CPC):
**G01N 27/327; G01N 27/416**

(86) International application number:
**PCT/JP2022/038668**

(87) International publication number:
**WO 2023/074455 (04.05.2023 Gazette 2023/18)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **28.10.2021 JP 2021176186**

(71) Applicant: **Panasonic Intellectual Property
Management Co., Ltd.
Osaka-shi, Osaka 540-6207 (JP)**

(72) Inventor: **TSUJI, Kiyotaka
Kadoma-shi Osaka 571-0057 (JP)**

(74) Representative: **Grünecker Patent- und
Rechtsanwälte
PartG mbB
Leopoldstraße 4
80802 München (DE)**

(54) **METHOD FOR CONTROLLING PROCESSING DEVICE, ENZYME ELECTRODE, AND PROCESSING DEVICE**

(57) The present disclosure provides a control method for a treatment device which is advantageous from the viewpoint of coping with change of a component contained in a treatment target while a reaction system including an oxidoreductase and a coenzyme is used. An enzyme electrode (1a) used in the control method for the treatment device according to the present disclosure includes a first conductor (11), a second conductor (12), and an enzyme portion (15). The second conductor (12) is electrically insulated from the first conductor (11). The enzyme portion (15) is fixed to a surface of the first conductor (11). The enzyme portion (15) includes the oxidoreductase and the coenzyme. The second conductor (12) has a surface to which only one enzyme selected from the group consisting of the oxidoreductase and the coenzyme is fixed, or to which a portion including both the oxidoreductase and the coenzyme is not fixed.

FIG. 3

```
              START
                |
S101   APPLY VOLTAGE BETWEEN FIRST CONDUCTOR 11 AND
       REFERENCE ELECTRODE 1b AND OBTAIN FIRST RELATIONSHIP R1
                |
S102   APPLY VOLTAGE BETWEEN SECOND CONDUCTOR 12 AND
       REFERENCE ELECTRODE 1b AND OBTAIN SECOND RELATIONSHIP R2
                |
S103   DETERMINE VOLTAGE Vt TO TREAT TARGET LIQUID 5
       TO BE TREATED
                |
S104   APPLY VOLTAGE Vt BETWEEN SECOND CONDUCTOR 12 AND
       REFERENCE ELECTRODE 1b AND MEASURE CURRENT
                |
S105   DETERMINE TARGET CURRENT Ia
                |
S106   APPLY VOLTAGE Vt TO FIRST CONDUCTOR 11
       AND START TREATMENT OF TARGET LIQUID 5
                |
S107   MEASURE CURRENT Imon FLOWING IN COUNTER ELECTRODE 1c
                |
S108        J1 ≤ J2 ?  ── No
                | Yes
S109   STOP APPLICATION OF VOLTAGE TO FIRST CONDUCTOR 11
                |
              END
```

**Description**

Technical Field

The present disclosure relates to a control method for a treatment device, an enzyme electrode, and the treatment device.

Background Art

A technique of utilizing a reaction system including an oxidoreductase and an electron transfer material is known up to now.

For example, Patent Literature (PTL) 1 discloses a method of measuring a glucose concentration by utilizing a reaction system including an enzyme and an electron transfer material. According to the disclosed method, glucose dehydrogenase (CyGDH) to which cytochrome C is bonded is used as the enzyme, and a Ru compound is used as the electron transfer material. In a glucose sensor including a reagent layer composed of a combination of the Ru compound and CyGDH, measurement of the glucose concentration reaches an endpoint in a short time upon the completion of a reaction with the glucose.

Citation List

Patent Literature

PTL 1: International Publication No. 03/106702

Summary of Invention

Technical Problem

The present disclosure provides a control method for a treatment device which is advantageous from the viewpoint of coping with change of a component contained in a treatment target while a reaction system including an oxidoreductase and a coenzyme is used.

Solution to Problem

The present disclosure provides a control method for a treatment device using a reaction system including an oxidoreductase and a coenzyme,

the treatment device comprising a reaction vessel in which a target liquid to be treated is contained, an enzyme electrode disposed inside the reaction vessel in contact with the target liquid, and a reference electrode disposed inside the reaction vessel in contact with the target liquid,
the enzyme electrode comprising:

a first conductor;
a second conductor electrically insulated from the first conductor; and
an enzyme portion fixed to a surface of the first conductor and including the oxidoreductase and the coenzyme, wherein the second conductor has a surface to which a portion including only one enzyme selected from the group consisting of the oxidoreductase and the coenzyme is fixed, or to which a portion including both the oxidoreductase and the coenzyme is not fixed,
the control method including:

applying a voltage between the first conductor and the reference electrode to obtain a first relationship between a first voltage value and a first current value corresponding to the first voltage value;
applying a voltage between the second conductor and the reference electrode to obtain a second relationship between a second voltage value and a second current value corresponding to the second voltage value; and
determining, based on the first relationship and the second relationship, a voltage to treat the target liquid.

Advantageous Effects of Invention

The control method for the treatment device according to the present disclosure is advantageous from the viewpoint of coping with change of a component contained in a treatment target while the reaction system including the oxidoreductase and the coenzyme is used.

Brief Description of Drawings

[Fig. 1A] Fig. 1A is a perspective view illustrating an example of an enzyme electrode according to an embodiment.
[Fig. 1B] Fig. 1B is a sectional view of the enzyme electrode taken along a cut surface denoted by a plane IB in Fig. 1A.
[Fig. 1C] Fig. 1C is a sectional view of the enzyme electrode taken along a cut surface denoted by a plane IC in Fig. 1A.
[Fig. 2] Fig. 2 illustrates an example of a treatment device according to the embodiment.
[Fig. 3] Fig. 3 is a flowchart illustrating an example of a control method for the treatment device according to the embodiment.
[Fig. 4] Fig. 4 is a flowchart illustrating another example of the control method for the treatment device according to the embodiment.
[Fig. 5] Fig. 5 is a graph illustrating time change of a current value in a working example.

Description of Embodiments

(Underlying Knowledge Forming Basis of the Present Disclosure)

Treating a treatment target, such as a food material, by a treatment device using a reaction system including an oxidoreductase and a coenzyme is practically conceivable. In this case, the necessity of preparing a target liquid to be treated, which contains the oxidoreductase, the coenzyme, and the treatment target, is eliminated because of using an enzyme electrode including the oxidoreductase and the coenzyme both immobilized on the enzyme electrode. Furthermore, work to separate and recover the oxidoreductase and the coenzyme from the treated liquid after treatment can also be omitted. It is considered that, in operation of the above-mentioned treatment device, a voltage is applied to the enzyme electrode, and whether to end the treatment is determined based on the magnitude of a current generated with the application of the voltage.

Studies conducted by the inventor suggest that types of components contained in the treatment target and concentrations of the individual components are not always constant, and that the types of the components contained in the treatment target and the concentrations of the individual components change depending on a type of the treatment target. It is therefore conceivable to, depending on the type of the treatment target, adjust operation conditions of the treatment device, such as a voltage applied to the enzyme electrode in the treatment device and the magnitude of a current indicating a guideline at which the treatment is to be ended.

As a result of intensive studies, the inventor has newly found that the treatment device can be operated depending on the treatment target with an improvement in configuration of the enzyme electrode. The inventor has accomplished, based on the above new knowledge, a control method for a treatment device, an enzyme electrode, and the treatment device according to the present disclosure.

(Embodiments of the Present Disclosure)

Embodiments of the present disclosure will be described below with reference to the drawings. It is to be noted that any embodiments described below represent general or specific examples. Numerical values, shapes, materials, constituent elements, arrangement positions of the constituent elements, forms of connection between the constituent elements, process conditions, steps, order of the steps, and so on, which are described in the following embodiments, are merely illustrative, and they are not purported to limit the scope of the present disclosure. Ones of the constituent elements in the following embodiments, those ones being not stated in an independent claim representing the most significant concept, are explained as optional constituent elements. Furthermore, the drawings are schematic views and are not always exactly drawn in a strict sense.

(Embodiment)

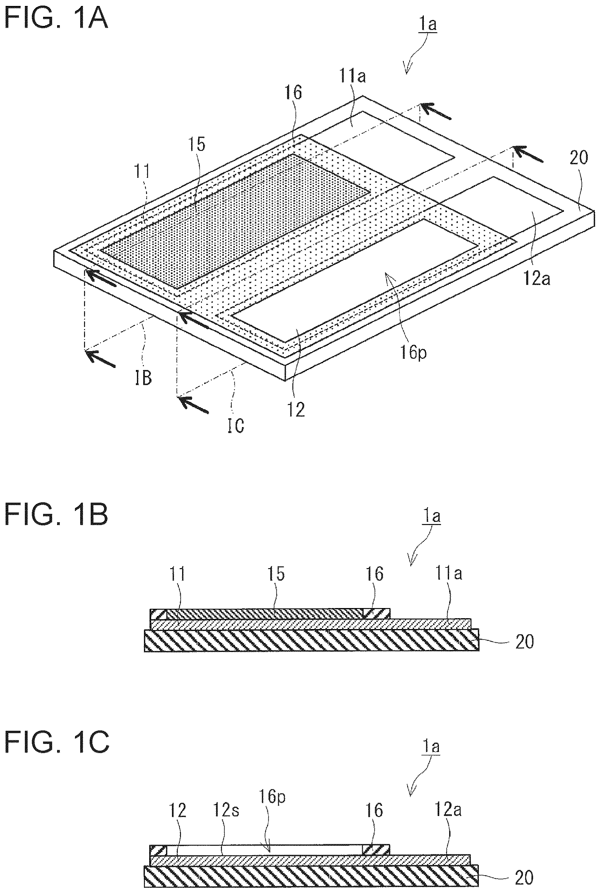

Fig. 1A is a perspective view illustrating an enzyme electrode 1a according to an embodiment. The enzyme electrode 1a includes a first conductor 11, a second conductor 12, and an enzyme portion 15. The second conductor 12 is electrically insulated from the first conductor 11. The enzyme portion 15 is fixed to a surface of the first conductor 11. Furthermore, the enzyme portion 15 includes an oxidoreductase and a coenzyme. A portion including both the oxidoreductase and the coenzyme is not fixed to a surface of the second conductor 12. In this case, at least part of the surface of the second conductor 12 may be exposed to the outside of the enzyme electrode 1a. A portion not including both the oxidoreductase and the coenzyme and having electron conductivity or ion conductivity may be fixed to at least part of the surface of the second conductor 12. A portion including only one enzyme selected from the group consisting of the oxidoreductase and the coenzyme may be fixed to the surface of the second conductor 12. In this case, for example, a portion including only one enzyme selected from the group consisting of the oxidoreductase and the coenzyme and having the electron conductivity or the ion conductivity may be fixed to the surface of the second conductor 12.

A conductive material forming the first conductor 11 and a conductive material forming the second conductor 12 are not limited to specific materials. The first conductor 11 and the second conductor 12 may each include at least one conductive material selected from the group consisting of a metal, an alloy, a conductive metal compound, and a conductive carbon material.

As illustrated in Fig. 1A, the enzyme electrode 1a includes, for example, a first contact portion 11a and a second contact portion 12a. The first contact portion 11a extends from the first conductor 11. The first contact portion 11a serves as a portion for electrical connection between the outside of the enzyme electrode 1a and the first conductor 11. The second contact portion 12a extends from the second conductor 12. The second contact portion 12a serves as a portion for electrical connection between the outside of the enzyme electrode 1a and the second conductor 12.

The enzyme electrode 1a includes, for example, a substrate 20 and an insulating film 16. The substrate 20 is, for example, an electrically insulating substrate such as a glass plate. The first conductor 11 and the second conductor 12 are formed, for example, on one principal surface of the substrate 20 in a layer shape apart from each other. In an example, the first conductor 11 and the first contact portion 11a are integrally formed, and the second conductor 12 and the second contact portion 12a are integrally formed. The insulating film 16 covers, for example, part of the substrate 20, part of the first conductor 11, and part of the second conductor 12. In an example, the first contact portion 11a and the second contact portion 12a are not covered by the insulating film 16.

Figs. 1B and 1C are sectional views of the enzyme electrode 1a taken along cut surfaces denoted by a plane IB and a plane IC in Fig. 1A, respectively. As illustrated in Figs. 1A and 1B, the enzyme portion 15 is formed, for example, on the surface of the first conductor 11 in a layer shape. The insulating film 16 covers, for example, part of the first conductor 11 except for a region in contact with the enzyme portion 15 on the first conductor 11. In a plan view of the enzyme electrode 1a, the enzyme portion 15 is surrounded by the insulating film 16.

The enzyme portion 15 includes, for example, a substance with ion conductivity. With that feature, predetermined ions can be easily supplied to the oxidoreductase and the coenzyme that are immobilized in the enzyme portion 15, and a reaction attributable to those enzymes is easier to occur. In an example, a region of the enzyme portion 15 fixed to the surface of the first conductor 11, the region including the oxidoreductase and the coenzyme, is covered by a layer including the substance with the ion conductivity.

The substance with the ion conductivity is not limited to a specific one. The substance with the ion conductivity is, for example, a polymer with a perfluoro side chain including a sulfonic group. With that feature, ions, such as protons, are easier to move more quickly and easily in the enzyme portion 15, and the reaction attributable to the oxidoreductase and the coenzyme is easier to generate. Nafion (registered trademark) can be used as the above-mentioned polymer.

As illustrated in Figs. 1A and 1C, the insulating film 16 has, for example, an opening 16p. The opening 16p is formed above the second conductor 12. An effective surface 12s of the second conductor 12, positioned open to the opening 16p in the plan view of the enzyme electrode 1a, is in contact with the outside of the enzyme electrode 1a. The effective surface 12s may be exposed to the outside of the enzyme electrode 1a or covered by a film other than the insulating film 16. In an example, the effective surface 12s may be covered by a film including only one enzyme selected from the group consisting of the oxidoreductase and the coenzyme. In this case, the effective surface 12s in contact with the above-mentioned film is regarded as being in contact with the outside of the enzyme electrode 1a.

The insulating film 16 is not limited to a specific one as far as it has predetermined electrical insulation properties. The insulating film 16 includes, for example, resin such as acrylic resin. When the enzyme electrode 1a includes the film including only one enzyme selected from the group consisting of the oxidoreductase and the coenzyme and formed on the surface of the second conductor 12, that film is regarded as a film different from the insulating film 16.

In an example, as illustrated in Fig. 1A, the enzyme portion 15 has a larger surface area S1 than an effective surface area S2 of the second conductor 12. With such a configuration, it is easier to provide a larger place where the reaction attributable to the oxidoreductase and the coenzyme occurs. The effective surface area S2 is, for example, a surface area of a portion of the second conductor 12, the portion being not covered by the insulating film 16. The effective surface area S2 is, for example, an area of the effective surface 12s.

A ratio S1/S2 of the surface area S1 to the effective surface area S2 is not limited to a specific value. The ratio S1/S2 may be, for example, greater than or equal to 1 and more specifically greater than or equal to 2. The ratio S1/S2 may be, for example, smaller than or equal to 10 and more specifically smaller than or equal to 5.

As illustrated in Fig. 1A, the enzyme electrode 1a has, for example, an integral structure including the first conductor 11, the second conductor 12, and the enzyme portion 15. The enzyme electrode 1a may be modified such that a first section including the first conductor 11 and the enzyme portion 15 and a second section including the second conductor 12 are constituted separately. In this case, the substrate 20 may be omitted.

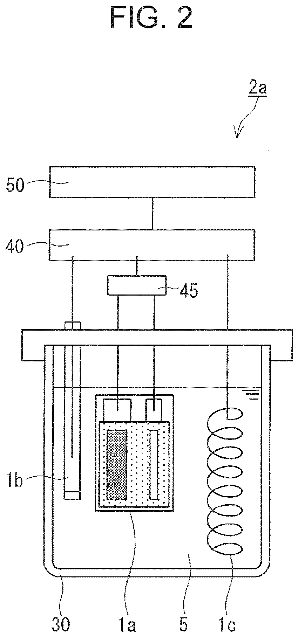

In an example, a predetermined treatment device can be provided by using the enzyme electrode 1a. Fig. 2 is a schematic view of a treatment device 2a according to the embodiment. The treatment device 2a includes a reaction vessel 30, the enzyme electrode 1a, and a reference electrode 1b. The enzyme electrode 1a is disposed inside the reaction vessel 30. The reference electrode 1b is disposed inside the reaction vessel 30.

In the treatment device 2a, the enzyme electrode 1a functions as an active electrode. As illustrated in Fig. 2, the treatment device 2a further includes a counter electrode 1c. The treatment device 2a is constituted as a three-electrode cell by the enzyme electrode 1a, the reference electrode 1b, and the counter electrode 1c.

As illustrated in Fig. 2, for example, a target liquid 5 to be treated can be put into the reaction vessel 30. In this case, the enzyme electrode 1a, the reference electrode 1b, and the counter electrode 1c are each disposed in contact with the target liquid 5 inside the reaction vessel 30.

As illustrated in Fig. 2, the treatment device 2a includes, for example, a voltage application device 40. The voltage application device 40 is, for example, a potentiostat. In this case, the enzyme electrode 1a, the reference electrode 1b, and the counter electrode 1c are electrically connected to an active electrode terminal, a reference electrode terminal, and a counter electrode terminal of the potentiostat, respectively.

The treatment device 2a includes, for example, a switch 45. The switch 45 switches between a first state and a second state. The first state is a state in which a voltage can be applied between the reference electrode 1b and the first conductor 11 of the enzyme electrode 1a. The second state is a state in which a voltage can be applied between the reference electrode 1b and the second conductor 12 of the enzyme electrode 1a. With the configuration described above, the first state and the second state can be switched to determine operation conditions of the treatment device 2a for treatment of the target liquid 5. The switch 45 may be operated manually or automatically.

The switch 45 is disposed, for example, between the active electrode terminal of the potentiostat and the enzyme electrode 1a.

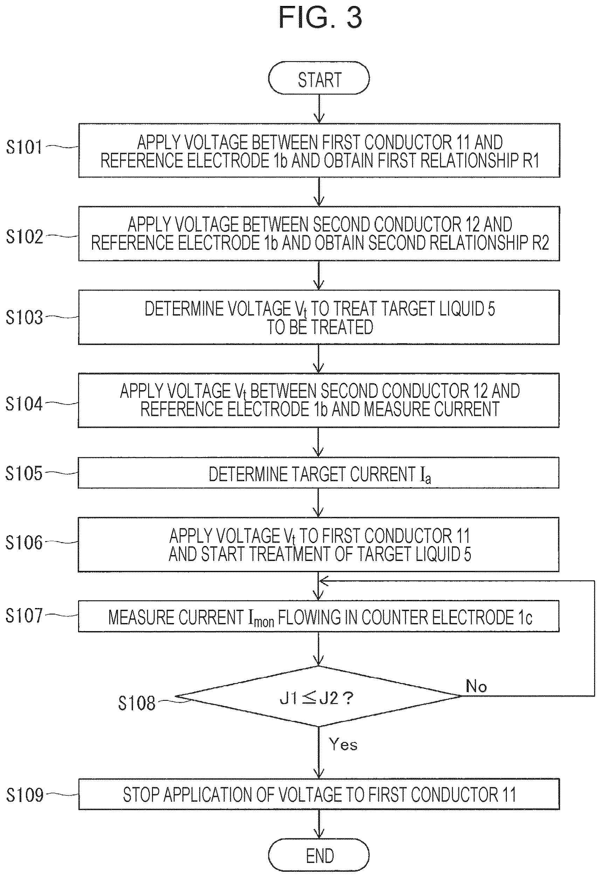

Fig. 3 is a flowchart illustrating an example of a control method for the treatment device 2a. In a state in which the target liquid 5 is put in the reaction vessel 30, a first relationship R1 is obtained in step S101 by applying, to the first conductor 11, a voltage that is given relative to the reference electrode 1b, and by applying a voltage to the counter electrode 1c according to a three-electrode method such that a current flows in the counter electrode 1c in an opposite direction to a current flowing in the first conductor 11, the former current having the same absolute value as the latter current. The first relationship R1 represents a relationship between a first voltage value and a first current value corresponding to the first voltage value. The first voltage value is a value of the voltage between the first conductor 11 and the reference electrode 1b. The first current value is, for example, a measurement value of the current in the counter electrode 1c. In step S101, the first relationship R1 is obtained, for example, by measuring the current in the counter electrode 1c while the voltage between the first conductor 11 and the reference electrode 1b is swept at a predetermined speed within a predetermined voltage range. Hereinafter, it is assumed, unless otherwise specified, that the voltage is given relative to the reference electrode 1b, and that a measurement system is in accordance with the three-electrode method.

As illustrated in Fig. 2, the treatment device 2a further includes, for example, a controller 50. The controller 50 is connected to the voltage application device 40 so as to be capable of controlling the voltage application device 40. The controller 50 is, for example, a digital computer in which a control program for the voltage application device 40 is stored and is executable. In step S101, the controller 50 obtains, for example, data indicating the first current value from the voltage application device 40. The controller 50 prepares data indicating the first relationship R1 by linking the obtained data with data indicating the first voltage value and stores the data indicating the first relationship R1 in a predetermined storage device.

Then, in step S102, a voltage is applied between the second conductor 12 and the reference electrode 1b, and a second relationship R2 is obtained. The second relationship R2 represents a relationship between a second voltage value and a second current value corresponding to the second voltage value. The second voltage value is a voltage value between the second conductor 12 and the reference electrode 1b. The second current value is, for example, a measurement value of the current in the counter electrode 1c. In step S102, the second relationship R2 is obtained, for example, by measuring the current in the counter electrode 1c while the voltage between the second conductor 12 and the reference electrode 1b is swept at a predetermined speed within a predetermined voltage range. The controller 50 obtains, for example, data indicating the second current value from the voltage application device 40. The controller 50 prepares data indicating the second relationship R2 by linking the obtained data with data indicating the second voltage value and stores the data indicating the second relationship R2 in a predetermined storage device.

Then, in step S103, a voltage Vt to be applied for treatment of the target liquid 5 is determined based on the first relationship R1 and the second relationship R2. Thus, the voltage to be applied for the treatment of the target liquid 5 can be adjusted according to change of a component contained in a treatment target in the target liquid 5.

A method of determining the voltage Vt is not limited to a specific one as far as the voltage Vt is determined based on the first relationship R1 and the second relationship R2. In step S103, for example, a voltage at which the current flowing in the counter electrode 1c increases abruptly in the first relationship R1 and the second relationship R2 with an increase in the first voltage value and the second voltage value is specified, and the specified voltage is determined as an upper limit value of the voltage Vt.

The voltage Vt is further determined based on, for example, a difference $\Delta I_{12}$ and a ratio $\Delta I_1/\Delta V_1$. The difference $\Delta I_{12}$ is a difference between the first current value in the first relationship R1 and a compensated value of the second current value in the second relationship R2. This difference indicates a difference between the first current value and the compensated value of the second current value, those first and second current values corresponding respectively to the first voltage value and the second voltage value that are the same. The compensated value of the second current value is determined by compensating for the second current value based on a relationship between the surface area S1 and the effective surface area S2. In an example, the compensated value of the second current value can be determined by multiplying the second current value by the ratio S1/S2. The ratio $\Delta I_1/\Delta V_1$ is a ratio of a change amount $\Delta I_1$ of the first current value to a change amount $\Delta V_1$ of the first voltage value. A treatment time for the target liquid 5 is more likely to be shorter in the determination of the voltage Vt by taking the difference $\Delta I_{12}$ into consideration. The treatment of the target liquid 5 is more likely to be stable in the determination of the voltage Vt by taking the ratio $\Delta I_1/\Delta V_1$ into consideration.

In step S103, for example, a voltage range where the difference $\Delta I_{12}$ is more than and equal to a predetermined lower limit value is specified within a voltage range where the voltage Vt is lower than and equal to its upper limit value. In addition, a voltage range where the ratio $\Delta I_1/\Delta V_1$ is less than and equal to a predetermined upper limit value is specified. The voltage Vt is determined from a range where the above-mentioned voltage ranges overlap.

Then, in step S104, the voltage Vt determined in step S103 is applied between the second conductor 12 and the reference electrode 1b, and a current value generated with the application of the voltage Vt is measured. For example, the current flowing in the counter electrode 1c is measured in step S104. Then, in step S105, a target current $I_a$ indicating a guideline at which the treatment of the target liquid 5 is to be ended is determined based on the current value measured in step S104. For example, a time average of the current value measured in step S104 is determined as the target current $I_a$. Thus, the target current $I_a$ is determined according to change of the component contained in the treatment target in the target liquid 5.

Then, as indicated in Steps S106 to S109, the target liquid 5 is treated by applying the voltage Vt to treat the target liquid 5 to the first conductor 11, and the application of the voltage Vt is stopped when a value of the current generated with the application of the voltage Vt and the target current $I_a$ satisfy a first condition.

In step S106, the voltage Vt to treat the target liquid 5 is applied to the first conductor 11, and the treatment of the target liquid 5 is started. Then, in step S107 corresponding to predetermined timing in a treatment period for the target liquid 5, a current $I_{mon}$ flowing in the counter electrode 1c is measured as the current value generated with the application of the voltage Vt. Then, in step S108, whether the current $I_{mon}$ and the target current $I_a$ satisfy the first condition is determined. For example, whether a current density J1 is less than or equal to a current density J2 or not is determined in step S108. The current density J1 is a value resulting from dividing the current $I_{mon}$ by the surface area S1 of the enzyme portion 15. The current density J2 is a value resulting from dividing the target current $I_a$ by the effective surface area S2 of the second conductor 12.

If a determination result in step S108 is negative, the control process returns to step S107 after the lapse of a predetermined time. Then, the current $I_{mon}$ flowing in the counter electrode 1c is measured again, and the determination in step S108 is executed.

If the determination result in step S108 is positive, the control process proceeds to step S 109 in which the application of the voltage to the enzyme electrode 1a is stopped, and a series of processing is ended.

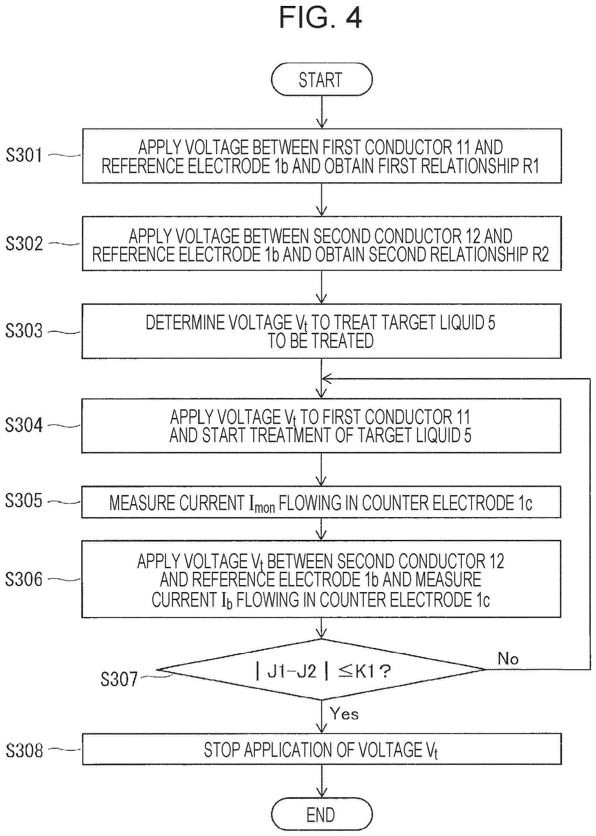

Fig. 4 is a flowchart illustrating another example of the control method for the treatment device 2a. Steps S301, S302, and S303 are executed in a similar manner to steps S101, S102, and S103, respectively.

As illustrated in Fig. 4, in steps S304 to S308, the target liquid 5 is treated by applying the voltage Vt determined in step S303 to the first conductor 11, and the application of the voltage Vt is stopped when a third current value and a fourth current value satisfy a second condition. The third current value is a value of the current generated when the voltage Vt is applied to the first conductor 11. The fourth current value is a value of the current generated when the voltage Vt is applied to the second conductor 12. Thus, timing to stop the treatment of the target liquid 5 can be adjusted in accordance with change of the component contained in the treatment target in the target liquid 5.

In step S304, the voltage Vt is applied to the first conductor 11, and the treatment of the target liquid 5 is started. Then, in step S305, the current $I_{mon}$ flowing in the counter electrode 1c is measured as the third current value in a state in which the voltage Vt is applied to the first conductor 11. Then, in step S306 after the lapse of a predetermined time, the first state is switched to the second state by the switch 45, the voltage Vt is applied between the second conductor 12 and the reference electrode 1b, and the treatment of the target liquid 5 is temporarily interrupted. In such a state, a current $I_b$ flowing in the counter electrode 1c is measured as the fourth current value. Then, in step S307, whether the third current value and the fourth current value satisfy the second condition is determined. In an example, whether an absolute value of a difference resulting from subtracting the current density J2 from the current density J1 is less than or equal to a predetermined value K1 or not is determined. The current density J1 is a value resulting from dividing the current $I_{mon}$ measured in step S305 by the surface area S1 of the enzyme portion 15. The current density J2 is a value resulting from dividing the current $I_b$ measured in step S306 by the effective surface area S2 of the second conductor 12.

If a determination result in step S307 is negative, the second state is switched to the first state by the switch 45, and the control process returns to step S304. On the other hand, if the determination result in step S307 is positive, the application of the voltage Vt to the enzyme electrode 1a is stopped, and a series of processing is ended.

K1 used in the determination of step S307 is not limited to a specific value. A ratio K1/J2 of K1 to J2 is, for example, about 0.1.

The oxidoreductase and the coenzyme are each not limited to a specific enzyme. When the target liquid 5 contains glucose, the oxidoreductase is, for example, glucose dehydrogenase (GDH). Furthermore, the coenzyme is, for example, nicotinamide adenine dinucleotide (NAD).

For example, a reaction expressed by the following formula (1) occurs in the treatment device 2a when the treatment target contained in the target liquid 5 is D-glucose, the oxidoreductase is GDH, and the coenzyme is NAD. GDH is involved as the oxidoreductase in this reaction.

$$\text{D-glucose} + \text{NAD}^+ \rightarrow \text{D-glucono-1,5-lactone} + \text{NADH} + \text{H}^+ \qquad \text{Formula (1)}$$

In the treatment device 2a, with the voltage Vt applied to the first conductor 11, reduced nicotinamide adenine dinucleotide (NADH) generated according to the Formula (1) is oxidized in the enzyme portion 15 of the enzyme electrode 1a serving as the active electrode. In this way, oxidized nicotinamide adenine dinucleotide (NAD$^+$) is generated. Since NAD$^+$ is generated with NADH oxidized continuously, glucose can be continuously decomposed by NAD included in the enzyme portion 15.

A material forming the counter electrode 1c is not limited to a specific one. In an example, the counter electrode 1c has a surface made of platinum. With that feature, electrons are easily released from the counter electrode 1c to maintain electrical neutrality of the target liquid 5 corresponding to oxidation of the coenzyme, and the treatment of the target liquid 5 can be efficiently performed. The material forming the surface of the counter electrode 1c may be gold (Au), any of carbon materials such as glassy carbon, graphite, and boron-doped diamond, or indium-tin oxide (ITO).

The shape of the counter electrode 1c is not limited to a specific one. The counter electrode 1c may have a linear, plate, or mesh shape or may be in the form of a fiber aggregate. From the viewpoint of efficiency in the treatment of the target liquid 5, it is advantageous that the counter electrode 1c has a large surface area.

The target liquid 5 may include food or a food material. The food or the food material contain a component as the treatment target. Concrete examples of the component are sugars such as glucose, and alcohols such as ethanol.

Working Example

The control method for the treatment device according to the present disclosure will be described in more detail in connection with a working example. It is to be noted that the control method for the treatment device and the treatment device according to the present disclosure are not limited to those ones described in the following working example.

A first conductive layer and a second conductive layer electrically insulated from each other were formed on a rectangular glass plate with a short side of 1 cm and a long side of 1.5 cm in a plan view by sputtering method in which a mask was used. A thickness of the glass plate was 0.7 mm. The first conductive layer and the second conductive layer each had a structure in which a chromium (Cr) film with a thickness of 50 nm and a gold (Au) film with a thickness of 200 nm were laminated. An insulating film covering part of the glass plate, part of the first conductive layer, and part of the second conductive layer was formed by using a material including photosensitive acrylic resin and having electrical insulation properties. On the other hand, one end portion of the first conductive layer and one end portion of the second conductive layer were not covered by the insulating film and were used as contact portions for electrical connection to the outside of an enzyme electrode. Moreover, a first portion occupying a predetermined part of a surface of the first conductive layer except for the one end portion of the first conductive layer and a second portion occupying part of a surface of the second conductive layer except for the one end portion of the second conductive layer were also not covered by the insulating film. Stated another way, the insulating film had a first opening corresponding to the first portion and a second opening corresponding to the second portion. In the plan view, the first portion had a rectangular shape with a short side of 4 mm and a long side of 8 mm. The second portion had a rectangular shape with a short side of 2 mm and a long side of 8 mm.

An enzyme-containing solution was prepared by dissolving glucose dehydrogenase (GDH) and reduced nicotinamide adenine dinucleotide (NAD) in a phosphate buffer with pH of 7.4. Glucose Dehydrogenase made by FUJIFILM Wako Pure Chemical Corporation was used as GDH. A concentration of GDH in the enzyme-containing solution was 5 g/milliliter (mL). Furthermore, β-nicotinamide adenine dinucleotide disodium (reduced form) made by NACALAI TESQUE INC.was used as NAD. A concentration of NAD in the enzyme-containing solution was $5 \times 10^{-3}$ mol/mL. A liquid film was formed by dripping 20 microliters ($\mu$L) of the enzyme-containing solution onto the first portion, and the formed liquid film was dried at room temperature (higher than or equal to 20 °C and lower than or equal to 25 °C). Another liquid film was formed by dripping 20 $\mu$L of a Nafion solution onto the dried liquid film of the enzyme-containing solution, and the formed liquid film was dried at the room temperature. A Nafion 117 containing solution provided by Sigma Aldrich was used as the Nafion solution. In such a manner, the enzyme electrode according to the working example, corresponding to the enzyme electrode 1a illustrated in Fig. 1A, was fabricated. In the enzyme electrode according to the working example, the second portion was exposed to the outside of the enzyme electrode.

A device according to the working example, corresponding to the treatment device 2a illustrated in Fig. 2, was fabricated by using the enzyme electrode according to the working example. A wiring was connected to each of the contact portion formed by the one end portion of the first conductive layer of the enzyme electrode and the contact portion formed by the one end portion of the second conductive layer thereof by applying a silver-containing paste, and those contact portions were embedded with silicone resin such that the contact portions were not in contact the target liquid. The wirings connected to the contact portions were connected to input terminals of a switch. The switch had two input terminals and one output terminal and was constituted to be able to manually switch connection of either one of the two input terminals to the one output terminal. The output terminal was electrically connected to an active electrode terminal of a potentiostat.

In the device according to the working example, a silver-silver chloride (Ag/AgCl) electrode was used as the reference electrode. An electrode formed by winding, into a coil shape, a platinum wire with a diameter of 0.5 mm and a length of 23 cm was used as the counter electrode. The reference electrode and the counter electrode were electrically connected to a reference electrode terminal and a counter electrode terminal of the potentiostat, respectively. Thus, the device according to the working example was constituted as a three-electrode cell.

An egg yolk and white of a commercially available chicken egg were manually separated, and an egg white liquid was obtained by manually stirring the separated egg white in a manner of cutting a highly viscous part by two chopsticks to such an extent as keeping the separated egg white from foaming, and then by removing a solid part with filtering through a gauze. A general egg white contains about 0.7% of glucose in terms of mass. A concentration of glucose in the general egg white is about $22 \times 10^{-3}$ mol/L. The obtained egg white liquid was put, as a target liquid to be treated, into a reaction vessel of the device according to the working example. A volume of the egg white liquid in the reaction vessel was 7.2 mL. The enzyme electrode, the reference electrode, and the counter electrode were in contact with the egg white liquid inside the reaction vessel.

By applying a voltage between the first portion of the enzyme electrode and the reference electrode, a first relationship between a first voltage value and a first current value corresponding to the first voltage value was obtained. Furthermore, by applying a voltage between the second portion of the enzyme electrode and the reference electrode, a second relationship between a second voltage value and a second current value corresponding to the second voltage value was obtained. The voltage to treat the egg white liquid was determined as +1.0 V based on the first and second relationships. In a state that the input terminal of the switch to which the wiring extending from the contact portion formed by the one end portion of the second conductive layer was connected was electrically connected to the output terminal of the switch, a direct current voltage of +1.0 V relative to a reference potential given at the reference electrode was applied to the enzyme electrode for 5 minutes. During a period in which the above-mentioned voltage was applied, a current in the counter electrode was measured and recorded every second. That measurement was performed at room temperature (higher than or equal to 15 °C and lower than or equal to 27°C), and the egg white liquid was stirred during the measurement by using a stirrer. An average value of the current in the counter electrode for the last one minute during the application period of the voltage was determined as the target current $I_a$. The target current $I_a$ in the working example was $5 \times 10^{-6}$ A.

Then, the switch was switched such that the input terminal of the switch to which the wiring extending from the contact portion formed by the one end portion of the first conductive layer was connected was electrically connected to the output terminal of the switch. The voltage of +1.0 V relative to the reference potential given at the reference electrode was continuously applied to the enzyme electrode, whereby the egg white liquid was treated. During a treatment period for the egg white liquid, a current $I_{mon}$ flowing in the counter electrode was measured and recorded. The treatment of the egg white liquid was performed at the room temperature (higher than or equal to 15 °C and lower than or equal to 27°C), and the egg white liquid was stirred during the treatment period for the egg white liquid by using the stirrer.

The application of the voltage to the enzyme electrode was stopped upon confirming that a current density calculated by dividing the current $I_{mon}$ by the surface area of the first portion had become smaller than or equal to a current density calculated by dividing the target current $I_a$ by the surface area of the second portion.

Fifty (50) $\mu$L of the egg white liquid sampled at predetermined timing during the period of the treatment of the egg white liquid was dripped onto a urine sugar examination zone of urine test paper New Uriace BT made by Terumo Corporation, and color change of the urine sugar examination zone was visually checked. A degree of glucose breakdown in the egg white liquid was confirmed based on the above-mentioned color change. A preliminary experiment using a test liquid with a previously adjusted glucose concentration was performed. As a result of the preliminary experiment, it was conformed that, if the glucose concentration in the test liquid was lower than or equal to $0.1 \times 10^{-3}$ mol/L, the color change of the urine sugar examination zone hardly occurred with the dripping of the test liquid. Furthermore, it was confirmed that, if the glucose concentration in the test liquid was higher than $0.1 \times 10^{-3}$ mol/L, color change of the urine sugar examination zone to dark blue was clearly discernible by a visual check.

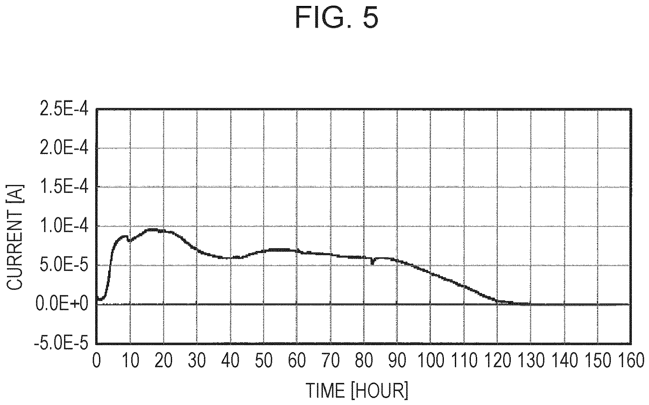

Fig. 5 is a graph illustrating a relationship between a time lapsed from the start of the application of the voltage to the enzyme electrode and a current in the counter electrode in the working example. As seen from Fig. 5, the current in the counter electrode gradually decreased after the lapse of about 90 hours from the start of the application of the voltage to the enzyme electrode. Furthermore, after the lapse of about 150 hours from the start of the application of the voltage to the enzyme electrode, the current density calculated by dividing the current $I_{mon}$ by the surface area of the first portion became lower than or equal to the current density calculated by dividing the target current $I_a$ by the surface area of the second portion. The glucose concentration in the egg white liquid sampled at a time after the lapse of every 50 hours from the start of the application of the voltage to the enzyme electrode was confirmed by using the urine test paper. As a result, glucose had been clearly detected in the egg white liquid until the lapse of 100 hours from the start of the application of the voltage to the enzyme electrode. At a time after the lapse of 150 hours from the start of the application of the voltage to the enzyme electrode, the color change of the urine test paper was not observed, and breakdown of glucose to a concentration lower than or equal to a detection limit in the target liquid was confirmed.

Industrial Applicability

The control method for the treatment device according to the present disclosure is useful in treatment of a target liquid to be treated, the treatment using a reaction system including an oxidoreductase and a coenzyme.

Reference Signs List

1a enzyme electrode
1b reference electrode
2a treatment device
5 target liquid to be treated
11 first conductor
12 second conductor
15 enzyme portion
30 reaction vessel
45 switch

Claims

1. A control method for a treatment device using a reaction system including an oxidoreductase and a coenzyme, wherein

the treatment device comprises a reaction vessel in which a target liquid to be treated is contained, an enzyme electrode disposed inside the reaction vessel in contact with the target liquid, and a reference electrode disposed inside the reaction vessel in contact with the target liquid,
the enzyme electrode comprises:

a first conductor;
a second conductor electrically insulated from the first conductor; and
an enzyme portion fixed to a surface of the first conductor and including the oxidoreductase and the coenzyme,

the second conductor has a surface to which a portion including only one enzyme selected from the group consisting of the oxidoreductase and the coenzyme is fixed, or to which a portion including both the oxidoreductase and the coenzyme is not fixed,
the control method comprising:

applying a voltage between the first conductor and the reference electrode to obtain a first relationship between a first voltage value and a first current value corresponding to the first voltage value; applying a voltage between the second conductor and the reference electrode to obtain a second relationship between a second voltage value and a second current value corresponding to the second voltage value; and determining, based on the first relationship and the second relationship, a voltage to treat the target liquid.

2. The control method for the treatment device according to claim 1, further comprising applying the voltage to treat the target liquid between the second conductor and the reference electrode and determining, based on a current value generated with application of the voltage, a target current indicating a guideline at which treatment of the target liquid is to be ended.

3. The control method for the treatment device according to claim 2, further comprising treating the target liquid by applying, to the first conductor, the voltage to treat the target liquid, and stopping the application of the voltage when a current value generated with the application of the voltage and the target current satisfy a first condition.

4. The control method for the treatment device according to claim 1, further comprising treating the target liquid by applying, to the first conductor, the voltage to treat the target liquid, and stopping application of the voltage when a third current value generated with the application of the voltage to the first conductor and a fourth current value generated with the application of the voltage to the second conductor satisfy a second condition.

5. The control method for the treatment device according to any one of claims 1 to 4, wherein the target liquid includes food or a food material.

6. An enzyme electrode used in the control method for the treatment device according to any one of claims 1 to 5, the enzyme electrode comprising:

a first conductor;
a second conductor electrically insulated from the first conductor; and
an enzyme portion fixed to a surface of the first conductor and including an oxidoreductase and a coenzyme, wherein the second conductor has a surface to which a portion including only one enzyme selected from the group consisting of the oxidoreductase and the coenzyme is fixed, or to which a portion including both the oxidoreductase and the coenzyme is not fixed.

7. The enzyme electrode according to claim 6, wherein the enzyme portion has a surface area which is larger than an effective surface area of the second conductor.

8. A treatment device comprising:

a reaction vessel;
an enzyme electrode according to claim 6 or 7, the enzyme electrode being disposed inside the reaction vessel, and
a reference electrode disposed inside the reaction vessel.

9. The treatment device according to claim 8, further comprising a switch that switches between a first state in which a voltage is applicable between the reference electrode and the first conductor and a second state in which a voltage is applicable between the reference electrode and the second conductor.

FIG. 1A

FIG. 1B

FIG. 1C

FIG. 2

# FIG. 3

START

S101 — APPLY VOLTAGE BETWEEN FIRST CONDUCTOR 11 AND REFERENCE ELECTRODE 1b AND OBTAIN FIRST RELATIONSHIP R1

S102 — APPLY VOLTAGE BETWEEN SECOND CONDUCTOR 12 AND REFERENCE ELECTRODE 1b AND OBTAIN SECOND RELATIONSHIP R2

S103 — DETERMINE VOLTAGE $V_t$ TO TREAT TARGET LIQUID 5 TO BE TREATED

S104 — APPLY VOLTAGE $V_t$ BETWEEN SECOND CONDUCTOR 12 AND REFERENCE ELECTRODE 1b AND MEASURE CURRENT

S105 — DETERMINE TARGET CURRENT $I_a$

S106 — APPLY VOLTAGE $V_t$ TO FIRST CONDUCTOR 11 AND START TREATMENT OF TARGET LIQUID 5

S107 — MEASURE CURRENT $I_{mon}$ FLOWING IN COUNTER ELECTRODE 1c

S108 — $J1 \leq J2$ ? —No

Yes

S109 — STOP APPLICATION OF VOLTAGE TO FIRST CONDUCTOR 11

END

# FIG. 4

START

S301 — APPLY VOLTAGE BETWEEN FIRST CONDUCTOR 11 AND REFERENCE ELECTRODE 1b AND OBTAIN FIRST RELATIONSHIP R1

S302 — APPLY VOLTAGE BETWEEN SECOND CONDUCTOR 12 AND REFERENCE ELECTRODE 1b AND OBTAIN SECOND RELATIONSHIP R2

S303 — DETERMINE VOLTAGE $V_t$ TO TREAT TARGET LIQUID 5 TO BE TREATED

S304 — APPLY VOLTAGE $V_t$ TO FIRST CONDUCTOR 11 AND START TREATMENT OF TARGET LIQUID 5

S305 — MEASURE CURRENT $I_{mon}$ FLOWING IN COUNTER ELECTRODE 1c

S306 — APPLY VOLTAGE $V_t$ BETWEEN SECOND CONDUCTOR 12 AND REFERENCE ELECTRODE 1b AND MEASURE CURRENT $I_b$ FLOWING IN COUNTER ELECTRODE 1c

S307 — | J1−J2 | ≤K1 ?        No

Yes

S308 — STOP APPLICATION OF VOLTAGE $V_t$

END

# FIG. 5

## INTERNATIONAL SEARCH REPORT

| International application No. |
| --- |
| **PCT/JP2022/038668** |

**A. CLASSIFICATION OF SUBJECT MATTER**

*G01N 27/327*(2006.01)i; *G01N 27/416*(2006.01)i
FI: G01N27/327 353S; G01N27/416 336M; G01N27/327 353F

According to International Patent Classification (IPC) or to both national classification and IPC

**B. FIELDS SEARCHED**

Minimum documentation searched (classification system followed by classification symbols)

G01N27/26-27/49, C12M1/00-3/10, C12Q1/00-3/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2012-132926 A (IKEDA SHOKKEN KK) 12 July 2012 (2012-07-12) paragraphs [0071]-[0087], fig. 4 | 6 |
| A | JP 2007-163268 A (CANON INC.) 28 June 2007 (2007-06-28) paragraphs [0123]-[0131], fig. 10-12 | 1-9 |
| A | JP 4-370755 A (NOK CORP.) 24 December 1992 (1992-12-24) claims | 1-9 |
| A | JP 2013-234857 A (TORAY IND., INC.) 21 November 2013 (2013-11-21) claims, paragraph [0011], fig. 7 | 1-9 |

☐ Further documents are listed in the continuation of Box C.  ☑ See patent family annex.

| * Special categories of cited documents: | |
| --- | --- |
| "A" document defining the general state of the art which is not considered to be of particular relevance | "T" later document published after the international filing date or priority date and not in conflict with the application but cited to understand the principle or theory underlying the invention |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **13 December 2022** | **27 December 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

INTERNATIONAL SEARCH REPORT
Information on patent family members

International application No.

**PCT/JP2022/038668**

| Patent document cited in search report | | | Publication date (day/month/year) | Patent family member(s) | | Publication date (day/month/year) |
|---|---|---|---|---|---|---|
| JP | 2012-132926 | A | 12 July 2012 | US 2011/0045513 A1 paragraphs [0167]-[0190], fig. 4 US 2011/0136158 A1 EP 2426200 A1 EP 2589659 A1 CA 2759911 A1 CA 2768045 A1 CN 102414319 A KR 10-2012-0023038 A CN 102559846 A | | |
| JP | 2007-163268 | A | 28 June 2007 | (Family: none) | | |
| JP | 4-370755 | A | 24 December 1992 | (Family: none) | | |
| JP | 2013-234857 | A | 21 November 2013 | (Family: none) | | |

Form PCT/ISA/210 (patent family annex) (January 2015)

17

**EP 4 425 163 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03106702 A